# EUROPÄISCHE PATENTSCHRIFT

(11) **EP 1 631 144 B1**
(45) Veröffentlichungstag und Bekanntmachung des Hinweises auf die Patenterteilung: **20.10.2010**
(21) Anmeldenummer: 04729644.7
(22) Anmeldetag: 27.04.2004
(51) Int. Cl.: A01N 39/02

(54) **POLYETHYLENGLYKOLESTER DER R-(+)-MCCPP-SÄURE**
POLYETHYLENE GLYCOL ESTERS OF R-(+)-MCPP ACID
ESTER DE POLYETHYLENE GLYCOL DE L'ACIDE R-(+)-MCCPP

(30) Priorität: 07.05.2003 DE 10320551
(43) Veröffentlichungstag der Anmeldung: 08.03.2006
(73) Patentinhaber: LANXESS Deutschland GmbH, 51369 Leverkusen (DE)
(72) Erfinder: UHR, Hermann, 51373 Leverkusen (DE); BRUNS, Rainer, 51373 Leverkusen (DE); ROTHER, Heinz-Joachim, 47803 Krefeld (DE); RENNER, Gerd-Friedrich, 51515 Kürten (DE)
(86) Internationale Anmeldenummer: PCT/EP2004/004414
(87) Internationale Veröffentlichungsnummer: WO 2004/098288

(56) Entgegenhaltungen:
- WO-A-95/06408
- DE-A- 4 412 330
- DE-B- 1 196 115
- US-A- 3 231 398
- LEHMANN,P.A.: "Stereoselectivity and affinity in molecular pharmacology. III. Structural aspects in the mode of action of natural and synthetic auxins" CHEM.-BIOL.INTERACTIONS, Bd. 20, 1978, Seiten 239-249, XP009038007

## Beschreibung

Die vorliegende Erfindung betriff neue, enantiomerenangereicherte Polyethylenglykolester der R-(+)-2-(4-Chlor-2-methylphenoxy)-propionsäure, deren beliebige Mischungen untereinander, Verfahren zu deren Herstellung und deren Verwendung als Durchwurzelungsinhibitoren.

Bei Baustoffen führt Wurzeldurchwuchs zu unerwünschten Materialschäden. Besonders plastische Materialien, wie Dichtungsmassen, Dachbahnen, aber auch Asphalt können von Pflanzenwurzeln zerstört werden. Das Eindringen von Wurzeln in Dichtungen von Kanälen und Abwasserleitungen, in Flachdachabdeckungen, in Bitumenisolierungen von Rohrleitungen, Brücken- und Wasserbauten sowie das Ein- und Durchwachsen von Wurzeln bei leichten Bitumen-Straßen sind allgemein bekannte Probleme. Undichtigkeiten, Korrosion, Schäden an Gebäuden, Straßen und Rohrleitungen können die Folge sein.

Um diese Schäden zu verhindern, wird z.B. in F. Hegemann, Abiogene Bitumenadditive, Bitumen-Teere-Asphalte-Peche 24, 105 (1973) beschrieben, Baustoffe mit wurzelwidrigen Wirkstoffen zu versetzen.

Aus der DE-A 1196115 sind Polyethylenglykolester der 2-(4-Chlor-2-methylphenoxy)-propionsäure bekannt, die eine Wurzelhemmende Wirkung haben.

In der DE-A 4412330 sind Verfahren zur Herstellung von Polyethylenglykolestem der 2-(4-Chlor-2-methylphenoxy)-propionsäure beschrieben, die von Polyethylenglykolen mit einer Molmassenverteilung von 170 bis 230 ausgehen, wobei die erhaltenen Produkte verbesserte wurzelinhibierende Eigenschaften aufweisen sollen.

Die WO 9506408 beschreibt die Verwendung von speziellen Estern der 2-(4-Chlor-2-methylphenoxy)-propionsäure zum Schutz von Bauwerken, Baustoffen und Isoliermassen gegen Durchwurzelung.

Außerdem ist bekannt, dass für herbizide Anwendungen im Pflanzenschutz, bei denen 2-(4-Chlor-2-methylphenoxy)-propionsäure eingesetzt wird, das R-(+)-Isomer deutlich wirksamer wie das S-(-)-Isomer oder die racemische Mischung ist (vgl. B. Åberg, Swedish J. agric. Res 1973, 3, 49).

Überraschenderweise wurden nun neue, enantiomerenangereicherte Polyethylenglykolester der R-(+)-2-(4-Chlor-2-methylphenoxy)-propionsäure der allgemeinen Formel (I) sowie deren Mischungen untereinander gefunden, die eine deutlich bessere Wirkung als Durchwurzelungs-inhibitoren haben als entsprechende Polyethylenglykolester der racemischen 2-(4-Chlor-2-methylphenoxy)-propionsäure oder Polyethylenglykolester der S-(-)-2-(4-Chlor-2-methylphenoxy)-propionsäure.

Gegenstand der vorliegenden Erfindung sind enantiomerenangereicherte Polyethylenglykolester der R-(+)-2-(4-Chlor-2-methylphenoxy)-propionsäure der allgemeinen Formel (I) und deren beliebige Mischungen untereinander 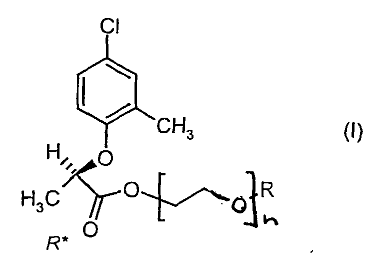 worin
- R: für H oder einen Rest der Formel 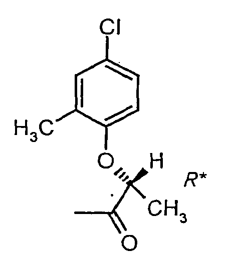 steht, und
- n: für eine ganze Zahl zwischen 1 und 20 steht, und
*R** die R-Konfiguration des chiralen Kohlenstoffatoms bezeichnet.

Enantiomerenangereichert im Sinne der Erfmdung bedeutet enantiomerenreine Verbindungen oder Mischungen von Enantiomeren einer Verbindung, in denen ein Enantiomer in einem Enantiomerenüberschuss, im Folgenden auch ee (enantiomeric excess) genannt, im Vergleich zum anderen Enantiomer vorliegt. Bevorzugt beträgt dieser Enantiomerenüberschuss 70 bis 100 % ee, besonders bevorzugt 80 % bis 100 Mol-% und ganz besonders bevorzugt 90 bis 100 Mol-%.

Enantiomerenangereichert im Sinne der Erfindung bezieht sich auf die Konfiguration des mit *R** bezeichneten Kohlenstoffatoms in Formel (I) und (III).

Weiterer Gegenstand der vorliegenden Erfindung ist die Verwendung von enantiomerenangereicherten Verbindungen der Formel (I) oder deren Mischungen untereinander zum Schutz von technischen Materialien, insbesondere von Bauwerken, Baustoffen und Bauhilfsmitteln gegen Ein- und Durchwachsen von Wurzeln.

Durch den Einsatz der erfindungsgemäßen enantiomerenangereicherten Polyethylenglykolester der R-(+)-2-(4-Chlor-2-methylphenoxy)-propionsäure und der erfindungsgemäßen Mischungen können die zur Verhinderung von Wurzeldurchwuchs notwendigen Einsatzmengen deutlich reduziert werden. Dieses ist aus ökologischen Gründen besonders zu befürworten, da man nur das wirksame Enantiomer einsetzt und unwirksame Verbindungen nicht in die Umwelt gebracht werden.

Aus dem Pflanzenschutz ist zwar bekannt, dass die herbizide Wirkung der freien R-(+)-2-(4-Chlor-2-methylphenoxy)-propionsäure höher wie die der racemischen 2-(4-Chlor-2-methylphenoxy)-propionsäure ist, eine Übertragung der herbiziden Effekte im Pflanzenschutz auf die durchwurzelungsinhibierenden Wirkungen z.B. in Folien oder Dachbahnen ist aber nicht naheliegend. Im Falle der herbiziden Anwendung werden unerwünschte Pflanzen zerstört, andererseits soll aber beim Einsatz von Durchwurzelungsinhibitoren nur das Einwachsen von Wurzeln verhindert werden. Durchwurzelungsinhibitoren zeigen in der Anwendung in Dichtungsmassen, Folien oder Dachbahnen keinerlei herbizide Wirkungen, so dass sie sich Beispielsweise gut als Unterlage und Wasserbarriere für Dachbegrünungen eignen.

Außerdem ist als überraschend zu bezeichnen, dass es bei den erfindungsgemäßen Verbindungen der Formel (I), entgegen dem allgemeinen Fachwissen, sowohl unter den Bedingungen der Herstellung als auch der Einarbeitung in technische Materialien, bei denen Temperaturen bis zu 200°C benötigt werden, nicht zu einer Racemisierung kommt.

Bevorzugt sind enantiomerenangereicherte Verbindungen der allgemeinen Formel (I), oder deren Mischungen untereinander, in welcher R die oben angegebene Bedeutung hat und n für eine ganze Zahl zwischen 2 und 10 steht.

Besonders bevorzugt sind enantiomerenangereicherte Verbindungen der allgemeinen Formel (I), oder deren Mischungen untereinander, in welcher R die oben angegebene Bedeutung hat und n für eine ganze Zahl zwischen 2 und 8 steht.

Des weiteren sind beliebige Mischungen von enantiomerenangereicherten Verbindungen der allgemeinen Formel (I) untereinander Gegenstand der Erfindung.

Im allgemeinen sind in diesen erfindungsgemäßen Mischungen die Einzelkomponenten der allgemeinen Formel (I) in Konzentrationen von jeweils bis zu 80 Gew.-% enthalten, wobei sich die Summe der Einzelkomponenten der Formel (I) auf 100 Gew.-% addiert. Bevorzugt sind erfindungsgemäße Mischungen in welchen die Einzelkomponenten der allgemeinen Formel (I) mit n = 3 bis 10 mit jeweils 0 bis 80 Gew.-% enthalten sind und die Einzelkomponenten der allgemeinen Formel (I) mit n = 1 sowie die Einzelkomponenten der allgemeinen Formel (I) mit n = 2 mit jeweils 0 bis 5 Gew.-% enthalten sind, wobei sich die Summe der Einzelkomponenten der Formel (I) auf 100 Gew.-% addiert.

Besonders bevorzugt sind erfindungsgemäße Mischungen von Verbindungen der allgemeinen Formel (I) worin die Einzelkomponenten der allgemeinen Formel (I) mit n = 3 bis 8 mit jeweils bis zu 80 Gew.-% enthalten sind, die Einzelkomponenten der allgemeinen Formel (I) mit n = 9 sowie die Einzelkomponenten der allgemeinen Formel (I) mit n = 10 mit jeweils 0 bis 10 Gew.-% enthalten sind und die Einzelkomponenten der allgemeinen Formel (I) mit n = 1 sowie die Einzelkomponenten der allgemeinen Formel (I) mit n = 2 mit jeweils < 1 Gew.-% enthalten sind, wobei sich die Summe der Einzelkomponenten der allgemeinen Formel (I) auf 100 Gew.-% addiert.

Die erfindungsgemäßen enantiomerenangereicherten Polyethylenglykolester der R-(+)-2-(4-Chlor-2-methylphenoxy)-propionsäure der allgemeinen Formel (I) können hergestellt werden, indem man Polyethylenglykol der allgemeinen Formel (II) 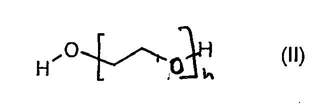 worin
n für eine ganze Zahl zwischen 1 und 20, bevorzugt für eine ganze Zahl zwischen 2 und 10 und ganz besonders bevorzugt für eine ganze Zahl zwischen 2 und 8 steht,
zusammen mit enantiomerenangereicherter R-(+)-2-(4-Chlor-2-methylphenoxy) propionsäure der Formel (III) 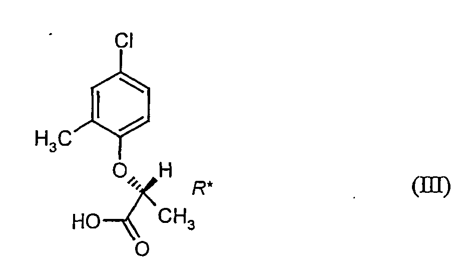 erhitzt und entstehendes Reaktionswasser abdestilliert. Gegebenenfalls kann das Abdestillieren von Reaktionswasser durch Anlegen von Vakuum unterstützt werden. Außerdem können gegebenenfalls auch Katalysatoren, wie beispielsweise Säuren eingesetzt werden.

Das molare Verhältnis zwischen R-(+)-2-(4-Chlor-2-methylphenoxy)-propionsäure der Formel (III) und dem Polyethylenglykol der Formel (II) beträgt im allgemeinen zwischen 1 : 1,2 und 2,2 : 1.

Bevorzugt liegt das molare Verhältnis zwischen R-(+)-2-(4-Chlor-2-methylphenoxy)-propionsäure und dem Polyethylenglykol der Formel (II) zwischen 1 : 1 und 2,1 : 1.

Die Temperaturen der Reaktion können in einem breiten Bereich variiert werden. Im allgemeinen liegen sie zwischen 100 und 250°C.

Bevorzugt liegen die Reaktionstemperaturen zwischen 130 und 230°C, besonders bevorzugt zwischen 150 und 210°C.

Durch Anlegen von Vakuum kann das Abdestillieren von Reaktionswasser unterstützt werden. Dabei ist es möglich bereits zu Begin der Reaktion Vakuum anzulegen oder erst in Laufe der Reaktion.

Im allgemeinen kann das Vakuum bis zu 0,5 mbar abgesenkt werden, bevorzugt wird ein Vakuum bis 1 mbar eingesetzt.

Die in die Reaktion eingesetzte enantiomerenangereicherte R-(+)-2-(4-Chlor-2-methylphenoxy)-propionsäure, sowie die Polyethylenglykole sind bekannt und kommerziell erhältlich.

Die Mischungen aus enantiomerenangereicherten Verbindungen der allgemeinen Formel (I) lassen sich entweder dadurch erhalten, dass man die enantiomerenangereicherten Einzelkomponenten der allgemeinen Formel (I) miteinander mischt oder bei der Herstellung statt reiner Polyethylenglykole, Mischungen aus Polyethylenglykolen der allgemeinen Formel (II) einsetzt.

Im allgemeinen können in den für die Synthese der Mischungen eingesetzten Polyethylenglykolgemischen die einzelnen Polyethylenglykole der allgemeinen Formel (II) in Konzentrationen jeweils bis zu 80 Gew.-% enthalten sein, wobei sich die Summe der Einzelkomponenten auf 100 Gew.-% addiert.

Bevorzugt werden Polyethylenglykolgemische eingesetzt, in welchen die einzelnen Polyethylenglykole der allgemeinen Formel (II) mit n = 3 bis 10 mit jeweils bis zu 80 Gew.-% enthalten sind und die einzelnen Polyethylenglykole der allgemeinen Formel (II) mit n = 1 und die der Formel (II) mit n = 2 mit jeweils 0 bis 5 Gew.-% enthalten sind, wobei sich die Summe der eingesetzten Polyethylenglykole der allgemeinen Formel (II) auf 100 Gew.-% addiert.

Besonders bevorzugt werden Mischungen von Polyethylenglykolen der allgemeinen Formel (II) eingesetzt, wobei die einzelnen Polyethylenglykole mit n = 3 bis 8 mit jeweils bis zu 80 Gew.-% enthalten sind, die Polyethylenglykole der allgemeinen Formel (II) mit n = 9 sowie die der allgemeinen Formel (II) mit n = 10 mit jeweils 0 bis 10 Gew.-% enthalten sind und die Polyethylenglykole der allgemeinen Formel (II) mit n = 1 sowie die der allgemeinen Formel (II) mit n = 2 mit jeweils < 1 Gew.-% enthalten sind, wobei sich die Summe der eingesetzten Polyethylenglykole der Formel (II) auf 100 Gew.-% addiert.

Die erfmdungsgemäßen enantiomerenangereicherten Polyethylenglykolester der R-(+)-2-(4-Chlor-2-methylphenoxy)-propionsäure der allgemeinen Formel (I) und Mischungen dieser enantiomerenangereicherten Polyethylenglykolester der R-(+)-2-(4-Chlor-2-methylphenoxy)-propionsäure der Formel (I) untereinander können insbesondere zum Schutz von Bauwerken wie z.B. Kanälen, Rohrleitungen, Häusern, Dächern, Strassen, Brücken- und Wasserbauten sowie von Baustoffen und Bauhilfsmitteln wie z.B. Zement, Kunststoff-Folien, Dachbahnen sowie von Baustoff-Zubereitungen wie z.B. Anstrichen, Dichtungs- und Isoliermassen gegen Ein- und Durchwachsen von Wurzeln verwendet werden.

Die erfindungsgemäßen Verbindungen der Formel (I) oder deren Mischungen können generell direkt auf die zu schützenden technischen Materialien aufgetragen oder mit diesen vermischt werden. So können z.B. Bauwerke direkt mit den erfindungsgemäßen Verbindungen der Formel (I) oder deren Mischungen behandelt werden, oder sie können mit Baustoff-Zubereitungen z.B. auf Basis von Bitumen oder Teerpechen, die eine oder mehrere erfmdungsgemäße Verbindungen der Formel (I) enthalten, behandelt werden. Die Baustoffe und Bauhilfsmitteln können z.B. mit den erfindungsgemäßen Verbindungen der Formel (I) oder deren Mischungen vermischt werden.

Die Baustoff-Zubereitungen können hergestellt werden, indem man eine handelsübliche Baustoff-Zubereitung, z.B. auf Basis von Bitumen oder Teerpechen, oder einen Anstrich oder eine Dichtungs- oder Isoliermasse mit einer oder mehreren erfindungsgemäßen Verbindungen der Formel (I) versetzt oder vermischt. Der Gehalt an enantiomerenangereichertem Wirkstoff der Formel (I) in den Baustoffen, Bauhilfsmitteln und Baustoff-Zubereitungen beträgt dabei im allgemeinen 0,01 bis 20 Gew.-%, bevorzugt 0,01 bis 5 Gew.-%, besonders bevorzugt 0,1 bis 2 Gew.-% bezogen auf die jeweilige Zubereitung bzw. den jeweiligen Baustoff.

### Beispiele:

### Beispiel 1

### (Diesters von Tetraethylenglykol mit R-(+)-2-(4-Chlor-2-methylphenoxy)-propionsäure)

60 g (0,28 Mol) R-(+)-2-(4-Chlor-2-methylphenox-y)-propionsäure, 24,7 g (0.13 Mol) Tetraethylenglykol und 0,5 g p-Toluolsulfonsäure wurden in einem Rundkolben mit Destillationsbrücke zusammengegeben und 6 h bei 180°C Innentemperatur gerührt. Hierbei destillierte entstehendes Reaktionswasser ab. Anschließend wurde die Temperatur auf 160°C abgesenkt und bei einem Druck von 80 mbar innerhalb von 6 h weiteres Reaktionswasser abdestilliert. Der Rückstand wurde über Kieselgel (Ethylacetat/ Toluol = 1:1) filtriert. Nach dem Abdampfen des Lösungsmittels erhielt man 71 g (68 % der Theorie) des Diesters von Tetraethylenglykol mit R-(+)-2-(4-Chlor-2-methylphenoxy)-propionsäure als hellrotes Öl. [α]_{D}²⁰ =+ 15,4° (c = 1, Ethanol) mit einem Enantiomerenüberschuss von > 95 Mol-%.

### Beispiel 2

### (Diesters von Tetraethylenglykol mit rac-2-(4-Chlor-2-methylphenoxy)-propionsäure/Vergleichsverbindung)

60 g (0,28 Mol) rac-2-(4-Chlor-2-methylphenoxy)-propionsäure, 24,7 g (0.13 Mol) Tetraethylenglykol und 0,5 g p-Toluolsulfonsäure wurden in einem Rundkolben mit Destillationsbrücke zusammengegeben und 6 h bei 180°C Innentemperatur gerührt. Hierbei destillierte entstehendes Reaktionswasser ab. Anschließend wurde die Temperatur auf 160°C abgesenkt und bei einem Druck von 80 mbar innerhalb von 6 h weiteres Reaktionswasser abdestilliert. Der Rückstand wurde über Kieselgel (Ethylacetat/ Toluol = 1:2) filtriert. Nach dem Abdampfen des Lösungsmittels erhielt man 82 g des Diesters von Tetraethylenglykol mit rac-2-(4-Chlor-2-methylphenoxy)-propionsäure als Öl. [α]_{D}²⁰ = + 0,1 ° (c = 1, Ethanol)

### Beispiel 3

719 g (2,18 Mol) R-(+)-2-(4-Chlor-2-methylphenoxy)-propionsäure und 385 g einer Glykolmischung, bestehend aus 33,6 % Triethylenglykol, 65,5 % Tetraethylenglykol und 1,2 % Pentaethylenglykol wurden in einem Planschliffreaktionsgefäß mit Destillationsbrüche vorgelegt und bei Normaldruck innerhalb von 4 h die Temperatur auf 200°C erhöht. Hierbei destillierte entstehendes Reaktionswasser mit leichten Verunreinigungen ab. Anschließend wurde zur Entfernung weiteren Reaktionswassers Vakuum (2 mbar) angelegt und weitere 16 h bei 200°C gerührt. Anschließend wurde abgekühlt und das Reaktionsprodukt über das Bodenventil abgelassen. Man erhielt 1010 g eines braunen Öls.

Zur Bestimmung der optischen Reinheit wurden 1,5 g NaOH in 10 ml Ethanol gelöst, mit 10 g des oben hergestellten Produktes versetzt und 2 h bei Raumtemperatur gerührt. Anschließend wurde auf 100 ml Wasser gegossen, 2 mal mit CH₂Cl₂ gewaschen und mit verdünnter HCl auf pH = 1-2 angesäuert. Das ölige Produkt wurde dann nochmals in 10 %iger NaOH gelöst, mit Toluol gewaschen und die Säure erneut durch Ansäuern mit HCl (10 %ig) ausgefällt. Nach trocknen im Vakuum wurde der Drehwert bestimmt: [α]_{D}²⁰ = + 15,3° (c = 1, CHCl₃). Die zur Reaktion eingesetzte R-(+)-2-(4-Chlor-2-methylphenoxy)-propionsäure wies einen Drehwert auf von [α]_{D}²⁰ = + 15,1° (c = 1, CHCl₃). Das erhaltene Produkt hatte einen Enantiomerenüberschuss von > 95 Mol-%.

### Beispiel 4

### (Vergleichsverbindung aus S-(-)-2-(4-Chlor-2-methylphenoxy)-propionsäure)

39 g (0,18 Mol) S-(-)-2-(4-Chlor-2-methylphenoxy)-propionsäure, 16,41 g (0.08 Mol) Tetraethylenglykol und 0,1 g p-Toluolsulfonsäure wurden in einem Rundkolben mit Destillationsbrücke zusammengegeben und 6 h bei 180°C Innentemperatur gerührt. Hierbei destilliert entstehendes Reaktionswasser ab. Anschließend wurde die Temperatur auf 160°C abgesenkt und bei einem Druck von 80 mbar innerhalb von 6 h weiteres Reaktionswasser abdestilliert. Der Rückstand wurde über Kieselgel (Ethylacetat/ Toluol = 1:1) filtriert. Nach dem Abdampfen des Lösungsmittels erhielt man 37,4 g (= 75 % der Theorie) des Diesters von Tetraethylenglykol mit S-(-)-2-(4-Chlor-2-methylphenoxy)-propionsäure als oranges Öl.

### [α]_{D}²⁰ 15,7° (c = 1, Ethanol)

### Beispiel 5

### (Temperung des Produktes aus Beispiel 3)

4 g des Produktes aus Beispiel 3 wurden 48 h in 40 ml o-Dichlorbenzol bei 180°C gerührt. Im Vakuum wurde die größte Menge an o-Dichlorbenzol abdestilliert. Anschließend gab man unter Kühlung eine Mischung von 0,6 g 50 %iger NaOH und 4 ml Ethanol zu und rührte 16 h bei Raumtemperatur. Dann wurde auf 40 ml Wasser gegeben und der pH Wert auf > 10 eingestellt. Man wusch zügig dreimal mit CH₂Cl₂ und säuerte die wässrige Phase mit HCl an, wobei sich ein Öl abschied, welches langsam auskristallisierte. Man saugte ab, trocknete im Vakuum, verrührte noch einmal mit n-Hexan und trocknete erneut im Vakuum. Man erhielt 1,52 g braun-beige Kristalle.
[α]_{D}²⁰ = + 15,3 ° (c = 1, Ethanol)

### Beispiel 6

### (Temperung des Produktes aus Beispiel 3 mit Zuschlagsstoffen)

4 g des Produktes aus Beispiel 3, sowie 2 g Kalksteinmehl, 2 g SBS-Kautschuk und 40 g o-Dichlorbenzol wurden 48 h bei 180°C gerührt.

Zunächst wurde mit 50 ml Methanol versetzt und von Unlöslichem abfiltriert und im Vakuum eingedampft. Zum Rückstand gab man anschließend unter Kühlung eine Mischung von 0,6 g 50 %iger NaOH und 4 ml Ethanol zu und rührte 16 h bei Raumtemperatur. Dann wurde auf 40 ml Wasser gegeben und der pH Wert auf > 10 eingestellt. Man wusch zügig dreimal mit CH₂Cl₂ und säuerte die wässrige Phase mit HCl an, wobei sich ein Öl abschied, welches langsam auskristallisierte. Man saugte ab, trocknete im Vakuum, verrührte noch einmal mit n-Hexan und trocknete erneut im Vakuum. Man erhielt 1,55 g beige Kristalle.
[α]_{D}²⁰ =+ 15,6° (c = 1, Ethanol)

### Beispiel 7

Die Verbindungen und Mischungen der Beispiele 1 und 2wurden gemäß DIN 4063 (Lupinentest) auf ihre Wirkung auf Hemmung der Durchwurzelung geprüft:

| **Komponente** | **Konzentration** | **Anzahl Wurzeleinwuchs** | **Anzahl Wurzeldurchwuchs** |
|---|---|---|---|
| Beispiel 1 | 0,1 % | ca. 40 | 0 |
| | 0,2 % | ca. 15 | 0 |
| | 0,5 % | 1 | 0 |
| Beispiel 2 | 0,1 % | ca. 40 | 1 |
| | 0,2% | ca. 40 | 0 |
| | 0,5 % | ca. 10 | 0 |
| | 1,0 % | 0 | 0 |
| Beispiel 4 | 0,1 % | > 50 | > 50 |
| (Vergleichsverbindung) | 0,2 % | >50 | > 0 |
| | 0,5 % | > 50 | > 50 |
| | 1,0 % | ca. 40 | ca. 40 |

## Patentansprüche

1. Enantiomerenangereicherte Polyethylenglykolester der R-(+)-2-(4-Chlor-2-methylphenoxy)-propionsäure der allgemeinen Formel (I) und deren beliebige Mischungen untereinander 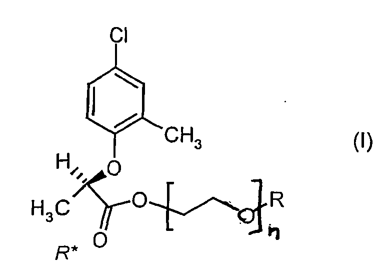 worin
R für H oder einen Rest der Formel 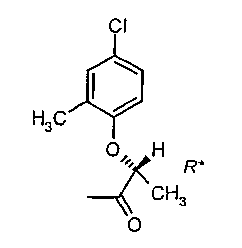 steht, und
n für eine ganze Zahl zwischen 1 und 20 steht, und
*R** die R-Konfiguration des chiralen Kohlenstoffatoms bezeichnet.

2. Enantiomerenangereicherte Verbindungen gemäß Anspruch 1, oder deren Mischungen untereinander, wobei in Formel (I) n für eine ganze Zahl zwischen 2 und 10 steht.

3. Mischungen enthaltend 0 bis 80 Gew.-% an mindestens zwei enantiomerenangereicherte Verbindungen der Formel (I) gemäß Anspruch 1, wobei sich die Summe der Einzelkomponenten der Formel (I) auf 100 Gew.-% addiert.

4. Verfahren zur Herstellung von enantiomerenangereichertem Polyethylenglykolester der R-(+)-2-(4-Chlor-2-methylphenoxy)-propionsäure der allgemeinen Formel (I) oder deren Mischungen untereinander gemäß Anspruch 1, **dadurch gekennzeichnet, dass** man mindestens einen Polyethylenglykol der allgemeinen Formel (II) 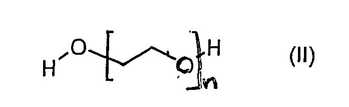 worin
n für eine Zahl zwischen 1 und 20 steht,
zusammen mit enantiomerenangereicherter R-(+)-2-(4-Chlor-2-methylphenoacy)-propionsäure der Formel 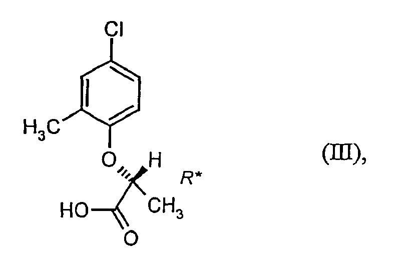 gegebenenfalls in Gegenwart von einem oder mehreren Katalysatoren erhitzt und entstehendes Reaktionswasser abdestilliert.

5. Verwendung von enantiomerenangereicherten Polyethylenglykolestern der R-(+)-2-(4-Chlor-2-methylphenoxy)-propionsäure der allgemeinen Formel (I) gemäß wenigstens einem der Anspruch 1 bis 3 zum Schutz von technischen Materialien gegen Ein- und Durchwachsen von Wurzeln.

6. Verwendung gemäß Anspruch 5, **dadurch gekennzeichnet, dass** es sich bei den technischen Materialien um Bauwerke, Baustoffe und Bauhilfsmittel handelt.

7. Mittel enthaltend enantiomerenangereicherte Verbindungen der Formel (I) gemäß wenigstens einem der Ansprüche 1 bis 3 sowie mindestens ein Lösungs- oder Verdünnungsmittel und gegebenenfalls weitere Verarbeitungshilfsmittel, Füll- und Zusatzstoffe.

8. Verfahren zum Schützen von technischen Materialien gegen Ein- und Durchwachsen von Wurzeln, **dadurch gekennzeichnet, dass** man enantiomerenangereicherte Verbindungen der Formel (I) gemäß wenigstens einem der Ansprüche 1 bis 3 entweder direkt auf das zu schützende technische Material aufträgt oder damit vermischt oder das technischen Material mit einem Mittel gemäß Anspruch 7 behandelt.

9. Technische Materialen enthaltend enantiomerenangereicherte Verbindung der Formel (I) gemäß wenigstens einem der Ansprüche 1 bis 3.

10. Verwendung eines Mittels gemäß Anspruch 7 zum Schutz technischer Materialien gegen Ein- und Durchwachsen von Wurzeln.

## Claims

1. Enantiomerically enriched R-(+)-2-(4-chloro-2-methylphenoxy)propionic acid polyethylene glycol esters of the general formula (I) and any of their mixtures with one another, 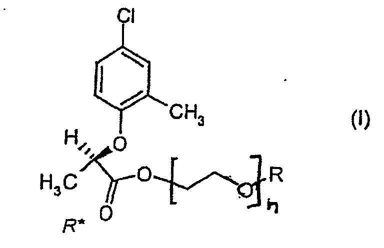 where
R represents H or a radical of the formula 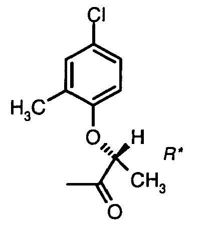 and
n represents an integer between 1 and 20 and
*R** denotes the R configuration of the chiral carbon atom.

2. Enantiomerically enriched compounds according to Claim 1 or their mixtures with one another, wherein, in formula (I), n is an integer between 2 and 10.

3. Mixtures comprising 0 to 80% by weight of at least two enantiomerically enriched compounds of the formula (I) according to Claim 1, wherein the total of the individual components of the formula (I) adds up to 100% by weight.

4. Method for the preparation of an enantiomerically enriched R-(+)-2-(4-chloro-2-methylphenoxy)propionic acid polyethylene glycol ester of the general formula (I) or mixtures thereof with one another according to Claim 1, **characterized in that** at least one polyethylene glycol of the general formula (II) 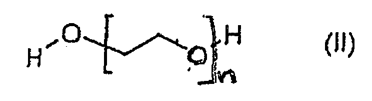 where
n represents a number between 1 and 20
is heated together with enantiomerically enriched R-(+)-2-(4-chloro-2-methylphenoxy)propionic acid of the formula 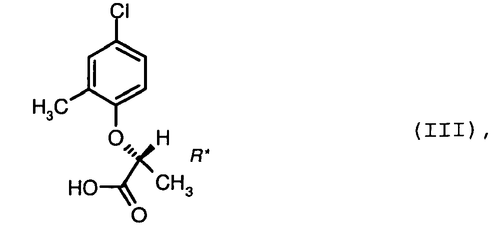 if appropriate in the presence of one or more catalysts and the water of reaction formed is distilled off.

5. Use of enantiomerically enriched polyethylene glycol esters of R-(+)-2-(4-chloro-2-methylphenoxy)propionic acid of the general formula (I) according to at least one of Claims 1 to 3 for the protection of industrial materials against root penetration thereinto and therethrough.

6. Use according to Claim 5, **characterized in that** the industrial materials are buildings, building materials and building auxiliaries.

7. Composition comprising enantiomerically enriched compounds of the formula (I) according to at least one of Claims 1 to 3 and at least one solvent or diluent and, if appropriate, further processing auxiliaries, fillers and additives.

8. Method for the protection of industrial materials against root penetration thereinto and therethrough, **characterized in that** enantiomerically enriched compounds of the formula (I) according to at least one of Claims 1 to 3 are either applied directly to the industrial material to be protected, or mixed therewith, or the industrial material is treated with a composition according to Claim 7.

9. Industrial materials comprising enantiomerically enriched compound of the formula (I) according to at least one of Claims 1 to 3.

10. Use of a composition according to Claim 7 for the protection of industrial materials against root penetration thereinto and therethrough.

## Revendications

1. Esters de polyéthylène glycol de l'acide R-(+)-2-(4-chloro-2-méthylphénoxy)-propionique énantiomériquement enrichis de formule générale (I) et leurs mélanges quelconques les uns avec les autres 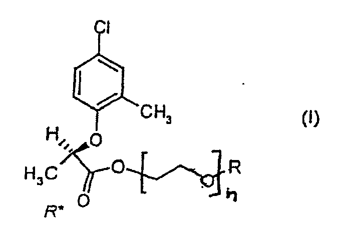 dans laquelle
R représente H ou un radical de formule 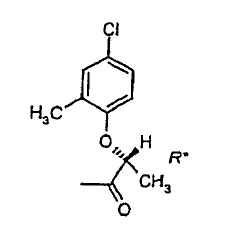 et
n représente un nombre entier compris entre 1 et 20, et *R** désigne la configuration R de l'atome de carbone chiral.

2. Composés énantiomériquement enrichis selon la revendication 1 ou leurs mélanges les uns avec les autres, dans lesquels n représente un nombre entier compris entre 2 et 10 dans la formule (I).

3. Mélanges contenant 0 à 80 % en poids d'au moins deux composés énantiomériquement enrichis de formule (I) selon la revendication 1, la somme des composants individuels de formule (I) atteignant 100 % en poids.

4. Procédé de fabrication d'esters de polyéthylène glycol de l'acide R-(+)-2-(4-chloro-2-méthylphénoxy)-propionique énantiomériquement enrichis de formule générale (I) ou leurs mélanges les uns avec les autres selon la revendication 1, **caractérisé en ce qu'**au moins un polyéthylène glycol de formule générale (II) 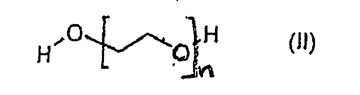 dans laquelle
n représente un nombre compris entre 1 et 20,
est chauffé avec l'acide R-(+)-2-(4-chloro-2-méthylphénoxy)-propionique énantiomériquement enrichi de formule 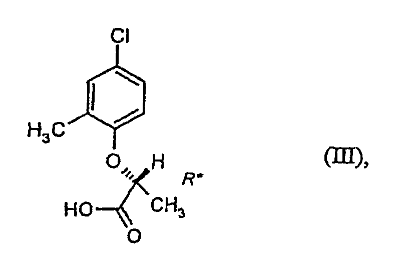 éventuellement en présence d'un ou de plusieurs catalyseurs et l'eau de réaction formée est éliminée par distillation.

5. Utilisation d'esters de polyéthylène glycol de l'acide R-(+)-2-(4-chloro-2-méthylphénoxy)-propionique énantiomériquement enrichis de formule générale (I) selon au moins l'une quelconque des revendications 1 à 3 pour la protection de matériaux techniques contre la croissance de racines dans et au travers de ceux-ci.

6. Utilisation selon la revendication 5, **caractérisée en ce que** les matériaux techniques sont des bâtiments, des matériaux de construction et des adjuvants de construction.

7. Agent contenant des composés énantiomériquement enrichis de formule (I) selon au moins l'une quelconque des revendications 1 à 3, ainsi qu'au moins un solvant ou diluant et éventuellement d'autres adjuvants de traitement, charges et additifs.

8. Procédé de protection de matériaux techniques contre la croissance de racines dans et au travers de ceux-ci, **caractérisé en ce que** des composés énantiomériquement enrichis de formule (I) selon au moins l'une quelconque des revendications 1 à 3 sont appliqués directement sur le matériau technique à protéger ou mélangés avec celui-ci ou le matériau technique est traité avec un agent selon la revendication 7.

9. Matériaux techniques contenant un composé énantiomériquement enrichi de formule (I) selon au moins l'une quelconque des revendications 1 à 3.

10. Utilisation d'un agent selon la revendication 7 pour la protection de matériaux techniques contre la croissance de racines dans et au travers de ceux-ci.
